# EUROPEAN PATENT SPECIFICATION

(11) **EP 4 418 019 B1**
(45) Date of publication and mention of the grant of the patent: **25.11.2026**
(21) Application number: 22880461.3
(22) Date of filing: 11.10.2022
(51) Int. Cl.: G01T 1/29, G01T 1/164

(54) **READOUT NETWORK TOPOLOGY FOR POSITRON EMISSION TOMOGRAPHY DEVICES WITH TIME OF FLIGHT**
AUSLESENETZWERKTOPOLOGIE FÜR POSITRONENEMISSIONSTOMOGRAFIEVORRICHTUNGEN MIT FLUGZEIT
TOPOLOGIE DE RÉSEAU DE LECTURE POUR DISPOSITIFS DE TOMOGRAPHIE PAR ÉMISSION DE POSITRONS À TEMPS DE VOL

(30) Priority: 12.10.2021 ES 202130959
(43) Date of publication of application: 21.08.2024
(73) Proprietor: General Equipment for Medical Imaging, S.A., 46022 Valencia (ES); Consejo Superior De Investigaciones Científicas - CSIC, 28006 Madrid (ES); Universitat Politècnica de València, 46022 Valencia (ES)
(72) Inventor: BARBERÁ BALLESTER, Julio, 46022 Valencia (ES); GONZÁLEZ MARTÍNEZ, Antonio Javier, 46022 València (ES); BENLLOCH BAVIERA, José María, 46022 València (ES); PAVÓN HERNÁNDEZ, Noriel, 46022 València (ES)
(74) Representative: Cueto, Sénida
(86) International application number: PCT/ES2022/070643
(87) International publication number: WO 2023/062263

(56) References cited:
- EP-A1- 3 399 345
- WO-A1-2019/228944
- US-A1- 2016 327 657
- US-A1- 2018 372 822
- US-A1- 2020 041 665
- US-A1- 2021 199 823
- GONZALEZ, A.J. ET AL.: "A PET Design based on SiPM and Monolithic LYSO Crystals: Performance Evaluation", IEEE TRANSACTIONS ON NUCLEAR SCIENCE, vol. 63, no. 5, 10 January 2016 (2016-01-10), pages 2471 - 2477, XP011625457, DOI: 10.1109/TNS.2016.2522179

## Description

### Field of invention

The present invention belongs to the field of medical devices for nuclear imaging. This kind of electronic design topology is intended to be a generic use in detectors for nuclear imaging, particularly in silicon photomultiplier individual photodetectors for Time-of-Flight applications.

### Background of the invention

Positron Emission Tomography (PET) involves administering living beings with trace amounts of biomolecules labelled with a positron-emitting radionuclide (radiotracers) and subsequent detection of the pairs of high energy gamma photons produced simultaneously following each positron-electron annihilation within the tissue.

The generated high energy photon pairs move in opposite directions, following straight lines, called Lines of Response (LOR), so that, it is possible to infer these LORs by detecting both photons' coordinates using gamma detectors surrounding the observed tissue. We also know that only the segment of the line that covers the space between the two gamma ray detectors touched by the pair of photons has a real probability to contain the point on the tissue where the positron-electron annihilation occurred.

So far, we have a collection of line segments containing information that allows us to infer radiotracer distribution within the tissue, using software reconstruction methods. This is what all PET technology usually does.

All PET systems include a timing coincidence filter to discriminate photons that are not clearly related to the same annihilation event, but the resolution of this timing filter (usually from a few ns to a few tens of ns) is not enough to extract more information. It is because in this time the light can travel a distance larger than the trans-axial dimension of the system.

Advanced PET systems called Time-of-Flight PET system (TOF-PET), are capable of measuring the time interval between the arrival of both high energy photons to their corresponding detectors with a high resolution (ranging from a few tens of picoseconds to a few hundreds of picoseconds), allowing to establish a specific short segment on the LOR (only a few cm) that has a real probability to contain the point on the tissue where the positron-electron annihilation occurred. The result is not a point on the LOR, but a segment, due to the uncertainty of the time measurement, characterized by the timing resolution of the system. In this case, the size of the data blocks required for the reconstruction process is considerably lower, what reduces the processing time, the proportion of invalid data and increases the precision and quality of the images. So, the accuracy of the measurement of the time arrival difference between both gamma rays involved in a single positron-electron annihilation is key to improve the next generation of Time-of-Flight PET detectors.

Another field of improvement is related to the gamma ray imaging detector technology, based on a detector tandem made of a scintillator detector coupled to a planar photodetector block made of a matrix arrangement of individual photodetectors.

In the text that follows (unless otherwise specified) we will use the word "photodetector" as well as "photodetector block" or "photodetector arrangement", to refer to a block that includes a plurality of individual photodetectors in a matrix arrangement, while, when referring to a photodetector formed by a single detector (for example an individual SiPM), we will explicitly indicate that it is an individual photodetector.

Nowadays, the older phototedector technology, based on photomultiplier tubes (PMT), is being substituted by advanced individual photodetectors based on solid state devices (for example SiPM) arranged in a matrix of (p x q) individual photodetectors. This allows to drastically reduce the cost per unit of photodetector area, but also important improvements in individual photodetectors reliability, uniformity, weight, electromagnetic immunity, etc. Arrangements of 4x4, 8x8, 16x16, 8x1, 16x1 SiPM, can usually be found in papers and are offered by manufacturers.

The drawback of this type of individual photodetectors is their high terminal capacitance which, together with their internal resistance, limits their response time. This is even worse if a large number of individual photodetectors are put together sharing the same connection points (parallel connection) since in this case, the total terminal capacitance is the sum of the individual capacitances of the individual photodetectors, provoking a worse time resolution of the system. So, regarding SiPMs based photodetectors, it is not possible to reduce the number of signals to process using the well-known reduction readout schemes trusted for the PMT photodetectors.

Therefore, to obtain the positioning and timing signals for the newest ToF-PET systems, the readout circuits usually deal with all SiPM signals separately at hardware level, and then, all these signals are digitized, generating a big amount of data to be later processed. It is very difficult to deal with the large number of components and circuits required if each SiPM should be acquired separately for energy and time response. If the time response is obtained after analog processing of the separated SiPM outputs, the time resolution is worsened unless very careful design be carried out across the whole analog design, to fine tune and equalize all the signal paths. This will result in an almost unattainable design if only a very small space is available. This is especially important if discrete electronics is used, although integrated electronics can benefit too from this arrangement. That is why, the creation of new reducing schemes become very useful to go forward with the possibility that new ToF-PET systems take advantages of the new SiPM photodetectors.

In order to reduce the effective capacitance of the individual photodetectors, for example SiPM, and its drawbacks, some authors used specific readout electronics to deal with it. US 2016/0327657 A1 describes a readout system that reduce the individual photodetector capacitance by a factor, coupling the individual SiPM photodetector to an analog front-end electronic through a transformer in a differential bootstrapping configuration. This could generally be achieved by connecting the individual photodetectors to any front-end electronics with a very low input impedance, not just by a transformer. Further, using transformers greatly complicates the possibility of miniaturization required for photodetectors blocks with many individual photodetectors such as those in the aforementioned arrangements.

They also split each individual photodetector signal by frequency band in order to use the high frequencies for timing purposes and the lower frequencies for energy and position determination, but they do not propose any reduction in the number of signals required, nor do they identify it as an issue to address, and even less do they disclose any scheme to achieve it.

Regarding the charge signals coming from each detector cell there are a lot of reduction schemes from the PMT era that are suitable for the SiPM arrangements era, provided that a few required modifications are made to avoid the drawback of the parallel addition of all SiPM capacitances, as we do in the present invention. Some examples of these reduction schemes can be found in our previous patents (US7476864B2, Pub. US20190004188A1). These are reduced to the specific scope of PET without ToF. Finally, US2018372822A1 discloses a multichannel ASIC for interfacing with an array of photodetectors in a PET imaging system, comprising a front-end circuit, a time discriminating circuit with a low input impedance amplifier generating a hit signal for timing pickoff, and an energy circuit generating summed energy and summed positional output signals.

### Description of the invention

In the present specification, whenever there is a reference to SiPMs as the most usual photodetectors, it should not be interpreted as a limitation of the invention to this type of photodetectors.

In an ideal PET made out of multiple detector modules, including at least one photodetector block per module it is required to obtain, with a sufficiently high precision some variables, such as the identification of the specific blocks receiving the annihilation photons interactions, the position in X and Y on the photodetector blocks surface, the Depth-of-Interaction within the scintillator detector volume, and the timestamps of such impact. The precision to obtain these variables depends on many characteristics related to the selected scintillator crystal type and geometry, the photodetector configuration and the associated electronics.

Due to the manufacturing process of photodetectors, such as SiPM, some spreading is found in different specific characteristics of the parts produced, even in a same batch, such as breakdown voltage, gain, time response, etc. In order to obtain the best uniformity, linearity and time accuracy all over the individual photodetectors surface, the circuits coupled to the individual photodetectors should deal with each of these deviations.

A readout electronic topology is provided in a variety of embodiments, as a signal reduction scheme, allowing the connection of a full arrangement of advanced individual photodetectors, such as of the individual SiPM photodetector type, containing a defined number of rows and columns, resulting in a single output, defined as a common timing signal used as the reference in the Time-of-Flight measurement of the entire photodetector block (simply called, the photodetector), maintaining the high time resolution required for ToF-PET systems. This represents a remarkable advance in reduction schemes of signals coming from a ToF-PET photodetector, to be processed and digitized, while ensuring a high image quality and a high performance of other measured parameters.

At the same time this arrangement allows to obtain individual uncoupled signals from each individual photodetector, and feeding those uncoupled signals to other suitable readout networks that collect all the charges of the photodetector arrangement, to accurately determine the event position and energy, without altering the high frequency components of the fast signals required for the determination of time references used in time-of-flight measurement.

These uncoupled output charge signals are compatible with other state of the art readout electronic circuits commonly used to reduce the number of charge signals, from (p x q) signals to (p + q) signals, and others, maintaining the capability to measure the event energy and position.

All the routes from each individual photodetector terminal to the circuit point where the single common timing signal is obtained, are tuned to synchronize the starting edges of all individual timing signal coming from any individual photodetector.

When a scintillation light pulse reaches the first, among any of the individual photodetectors, such as a SiPM, the starting edge of the highest frequency component of the signal generated passes through a very high-frequency band-pass filter formed by the active individual photodetector, a bias filter and an amplifier used to boost the timing output signal. The filter frequency band is close to the highest frequency components of the photodetector pulse.

A parallel set of different filters (charge filters) with a significant lower frequency band (a frequency at least 10 times lower than previous one) is used to connect the photodetector signals apart, to another readout network designed to extract the charges involved in the events at any place inside the photodetector.

The signals of both filters do not interfere each other due to its frequency separation, allowing us to have both, an early accurate single timing signal of an event occurred at the position of any individual photodetector in the arrangement, and the belated charge signals generated at each one of the individual photodetectors.

The present invention has a first object a readout network topology, as defined in claim 1, for positron emission tomography (PET) with time of flight (TOF) capabilities to extract position and time information in the area of gamma-ray detection comprising:
- a plurality of individual photodetectors arranged in p x q matrix configuration on an electronic Printed Circuit Board (PCB) to conform a photodetector capable to process the light arriving to the matrix area and providing information to determine spatial coordinates and timing signal, each individual photodetector having an anodic terminal and a cathodic terminal,
- a connection of all similar terminals of one of the two types of photodetector terminals (either the anodic ones or the cathodic ones) to a Single Junction at the PCB, which further splits into two routes,
   - the first one of these routes connects the Single Junction, by means of a specific impedance, to the proper source polarity, wherein "proper polarity" means: Vbias if the wired terminals to the Single Junction are the cathodes or GND if the wired terminals to the Single Junction are the anodes, wherein the specific impedance, either Za (if the wired terminals are the anodes) or Zk (if the wired terminals are the cathodes), is working as part of the photodetector bias circuit, and its resistance is set to be less than 10% compared to the resistance of the rest of the route from the Single Junction to GND and,
   - the second one of these routes feeds the unified signal created at the Single Junction, into a fast amplifier, in order to obtain a single timing signal regarding the whole photodetector, no matter which specific individual photodetector it comes from,
- an individual connection of all the photodetector terminals, different from those ones connected to the Single Junction, by means of two separate filtering circuits for each photodetector terminal,
   - the first filtering circuits named bias filters (TBF or BBF), complete the bias circuits for all individual photodetectors, connecting, by means of them, the individual photodetector terminals to the proper source polarity, wherein "proper" means that it is Vbias if the photodetector terminal is the cathode or GDN if the photodetector terminal is the anode and,
   - the second filtering circuit named charge filters function as output signal filters, to obtain individual current signals from each individual photodetector, representing the quantity of light captured by each individual photodetector in the arrangement, giving rise to uncoupled Xᵢ signals.

The bias filters TBF or BBF, as well as the charge filters CF, could be as simple as RC filters, but they should be trimmed to operate at frequency bands separated enough to avoid signal interference between the timing signal and the charge signals.

According to particular embodiments, in the readout network topology, defined above, the time reference signal is the sum of all the cathode terminals of the photodetector in the Single Junction (K).

According to other particular embodiments, in the readout network topology defined above, the time reference signal is the sum of all the cathode terminals of the photodetector at the Single Junction (Pzk), including the delay lines routed on the printed circuit board.

According to particular additional embodiments, in the readout network topology defined above, the timing signal is the sum at the Single Junction (A) of all photodetector anodic terminals.

According to particular additional embodiments, in the readout network topology defined above, the timing signal is the result of the differential signal between the Single Junction (K) of all photodetector cathode terminals and the Single Junction (Pza) of all photodetector anode terminals indirectly wired to Pza.

According to more particular additional embodiments, in the readout network topology defined above, the timing signal is the result of the differential signal between the Single Junction (A) of all photodetector anode terminals and the Single Junction (Pzk) of all photodetector cathode terminals indirectly wired to Pzk.

According to more particular additional embodiments, in the readout network topology defined above, the timing signal is the result of the Single Junction (Pzk) due to the sum of all photodetector cathode terminals indirectly wired to Pzk.

According to particular additional embodiments, in the readout network topology defined above, the timing signal is the result of the Single Junction (Pza) due to the sum of all photodetector anode terminals indirectly wired to Pza.

According to preferred embodiments in readout network topology according to any of the preceding claims, the individual photodetectors are single SiPM devices.

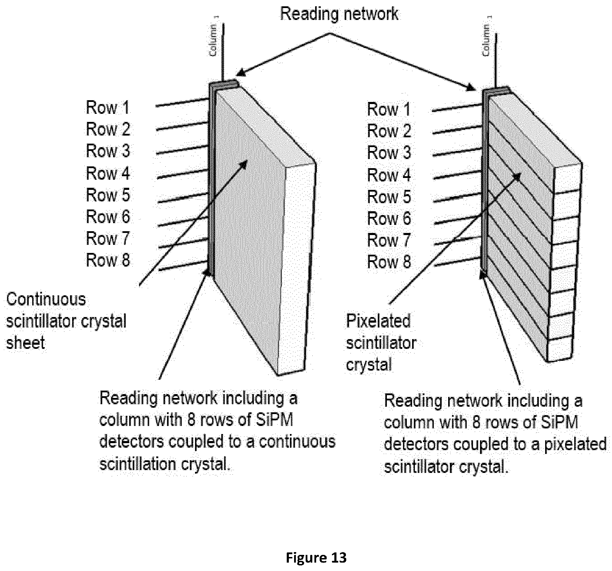

The present invention has a second object a nuclear detector module capable for ToF-PET applications, comprising one or more readout network topologies, having each one a photodetector as defined herein above. (Figure 13)

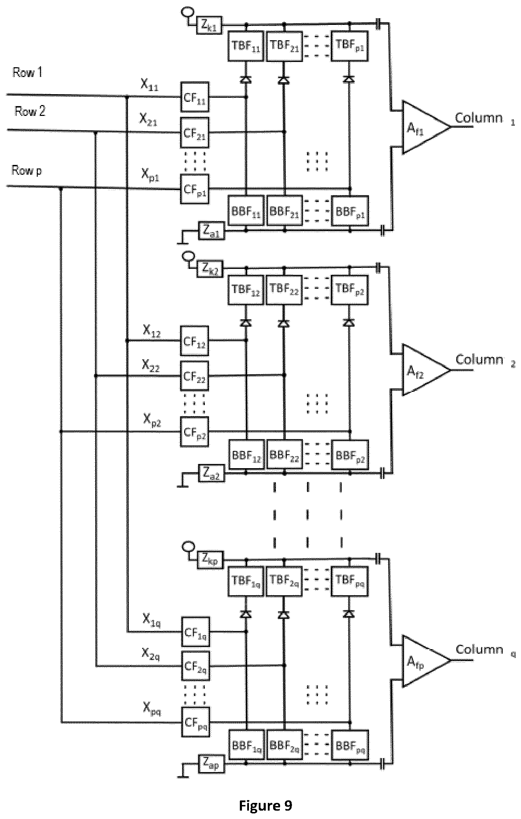

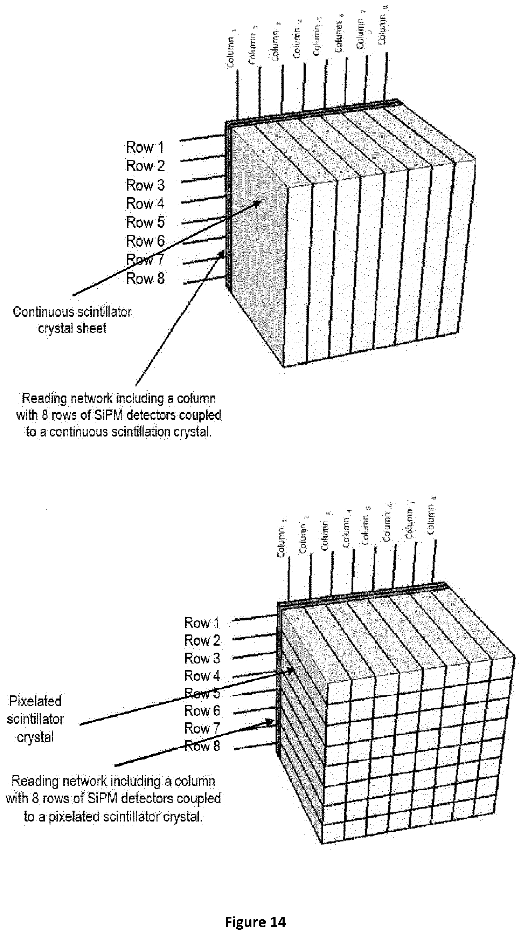

The nuclear detector module (Figure 14) can have a plurality of readout networks arranged, having one column, and a number of p rows, such that the different timing signals obtained from each readout networks are connected to a charge readout schemes in order to obtain energy and position information regarding the plurality of readout networks, (Figure 9).

The nuclear detector module can further comprise:
- a scintillator crystal part facing the photodetector.
- a housing to protect the module from external light and mechanical stress,
- an analog to digital conversion electronics capable of processing all positioning and energy signals obtained, and transmitting these through a high-speed computer interface to be processed on the fly, or stored in a computer disk, to be later processed, as well as preserving the timing signal.

Since this readout network topology can be piled up, a variety of different detector modules configurations can be manufactured that include a plurality of readout networks according to the present invention, that can be accommodated too in an upper level matrix arrangement, wherein matrix intersections are the readout networks, instead of the individual photodetectors that are the intersections in the readout network individual photodetectors.

The stacking of several readout networks of this topology also constitutes a readout network of this topology, although it is presented as a higher-level or stacked structure, while from the point of view of the stacked readout network, its constituent readout networks, are lower level readout networks. This can be seen as a fractal structure, as it describes a structure that maintains the same form and functionality at different levels of integration of its stackable components.

According to particular embodiments, (Figure 9) in a higher-level readout network, q lower-level readout networks are stacked, each of which has a single column and a number of rows (p), to form the higher level readout network. The stacked readout network provides q outputs of time signals that can be acquired separately or through a load network to obtain positioning along the columns, while the number of load signals Xᵢ, remains as it was before stacking of the lower level networks, since they overlay (join and sum) all the loading signals of each row along all the columns and can be acquired individually or with any loading scheme, to obtain the position along the p rows, in the same way as in the variants without stacking of readout networks. For example, the overlap in row 2 is as follows: ΣX₂; (j = 1..q).

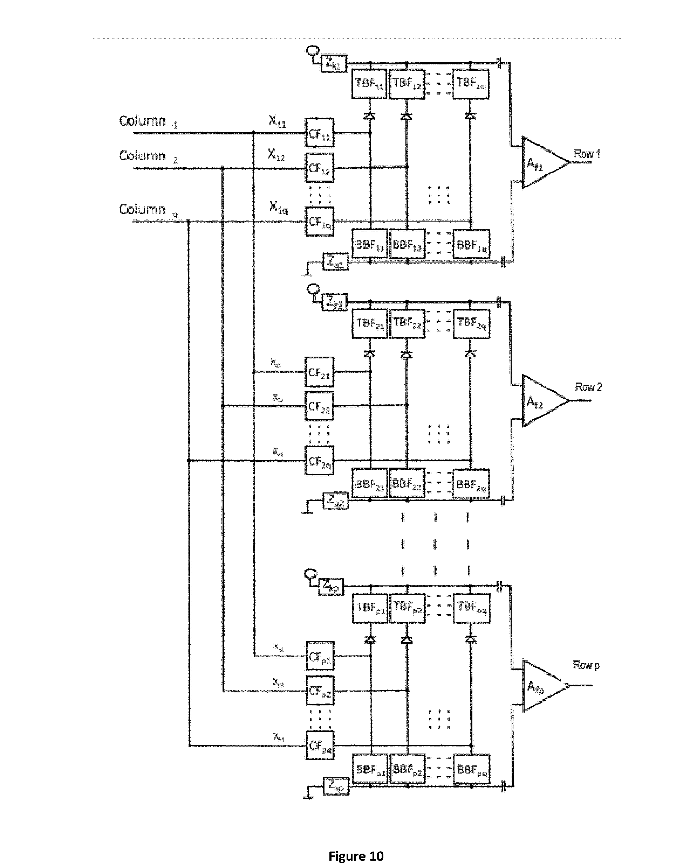

The arrangement of rows and columns is an arbitrary convention, so rows and columns are interchangeable. According to additional particular embodiments, (Figure 10) in a higher-level readout network, p lower-level readout networks, each of which has a row and a number of columns (q), are stacked to form the higher level readout network. The stacked readout network provides p time signal outputs that can be acquired separately or through a load network to obtain positioning along the rows, while the number of load signals Xᵢ, remains as it was before the stacking of the lower level networks, since they overlay (join and sum) all the loading signals of each column along all the rows and can be acquired individually or with any loading scheme to obtain the position along the rows. columns, in the same way as in the variants without stacking of readout networks. For example, the overlap in column 2 is as follows: ΣXᵢ₂ (i = 1..p).

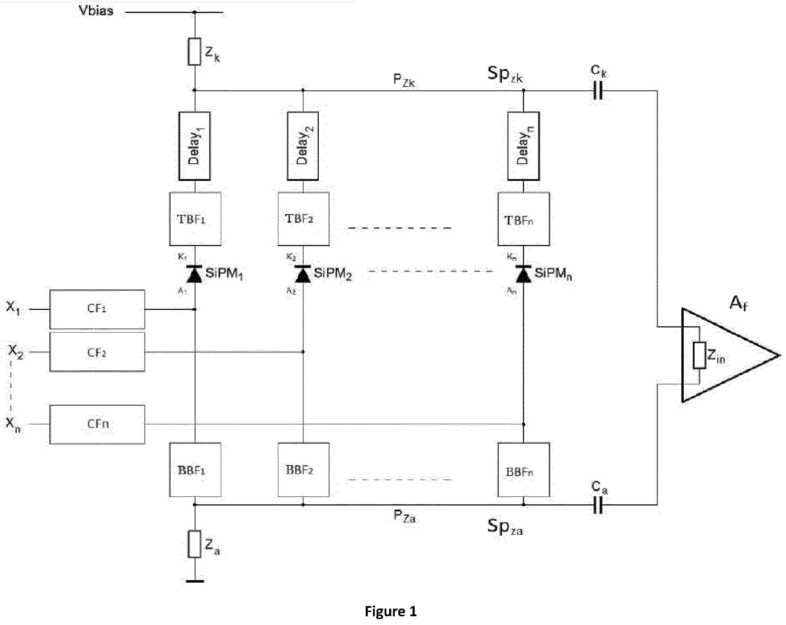

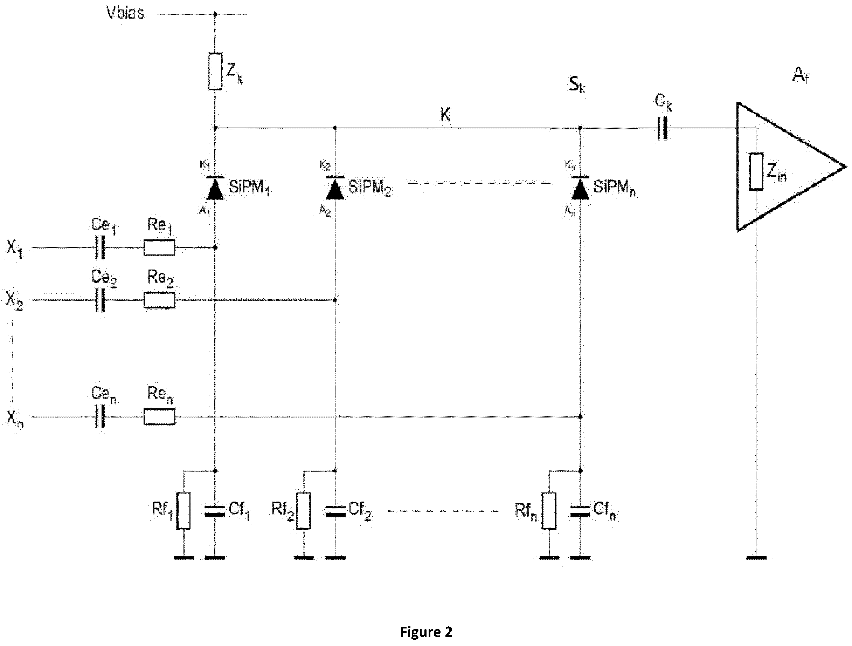

Each of the generic configurations of Figure 9 and Figure 10 can be divided into 6 embodiments, if specific configurations of readout networks are used, in the same way that the configurations of figures 2, 4, 5, 6, 7 and 8 are derived from Figure 1.

The present invention has a third object a ToF-PET System comprising:
- a plurality of detector modules as the ones described above, arranged in a mechanical gantry, having a symmetrical configuration, with all modules surrounding and facing the volume of detection that can be covered by the detector modules, surrounding a free volume enough to accommodate a living being or tissue for ToF-PET imaging, and including processing software and synchronization electronics to enable the detection of all signals coming from any module, being charge signals or timing signals.
- a coincidence processing electronic module capable to process the timing signals coming from a plurality of modules as described above, to provide the proper matching between multiple combinations of detector modules, allowing to effectively process as a single block, all signals related to any single PET event, no matter which detector module they come from, as well as capable to digitize and transmit all the timing related signals through a high-speed computer interface to be processed on the fly, or stored in a computer disk, to be later processed, including time of flight information of all detected events.

The ToF-PET System can further comprise a software module capable of processing all the data stored in a computer or being received in real time, to provide high quality ToF-PET images, using any computer or digital interface.

The readout network topology for positron emission tomography (PET) with time of flight (TOF) capabilities of the present invention makes it possible to get the belated individual charge information from each photodetector, without affecting the early, fast-transition signals coming from the photodetector parallel common output.

The description and figures essentially describe the topology by means of an undefined number of basic cells and the other components that are not bound to the cell.

All the components bound to the cell will appear below with an index "i", wherein i ranges from 1 to n, being n = p x q, being p the rows and q the columns of the matrix arrangement, and being p and q integers higher than 0.

Often, photodetector signals are first digitized for later processing, but, as better resolution systems require increasing the number of individual detectors in the matrix, this also provokes the increasing of the number of electronic channels, which limits the minimum size achievable photodetector block.

This invention has the advantages of a significant reduction in components, reduction of circuit complexity, reduction of parasitic capacitance and energy consumption.

The readout network topology of the invention uses the typical bias circuit of a photodetector arrangement (a full matrix with p rows and q columns), with some specific modifications, to also extract a unique, accurate and phase-tuned timing signal, regarding the event at any position of the whole photodetector arrangement, no matter what individual photodetector it came from.

The readout network topology here described, is characterized by being properly trimmed and connected to a plurality of light detectors in a matrix configuration, such as SiPM arrangement, extracting timing and charge signals, with a significant reduction in components, circuit complexity, parasitic capacitance and energy consumption, which enables it for ToF-PET applications.

The present invention, as defined in claim 14, also has as an object a process to obtain the time and position information from the signals coming from a ToF-PET photodetector, in a detector module of a PET device, the process comprising:
- arranging a scintillator crystal in a detector module
- arranging a readout network topology as the one defined in the present invention,
- entering a gamma ray into the module and impinging the scintillator crystal volume, creating a light beam which spreads out everywhere, including the readout network photodetector area,
- entering the light beam coming from a scintillator crystal into the readout network photodetector area,
- converting the light beam into a spreaded distribution of electrical signals coming from the whole photodetector area,
- bringing the first signal detected from any part of the photodetector to a fast amplifier to point it out as the output timing signal, representing the exact instant of the arrival of the gamma ray to the scintillator crystal volume,
- bringing every one of the electrical signals coming from the whole photodetector area, by means of charge filters to provide uncoupled charge signal outputs compatible with readout electronic circuits commonly used to extract position and energy of the gamma ray.

The process can further comprise handling data from at least two modules, providing information according to claim x obtaining the ToF lines of response required to create a ToF-PET image.

### Brief description of the drawings.

Figure 1 shows a general diagram of a circuit design topology according to the invention.
Figure 2 illustrates a circuit design embodiment where the timing signal is obtained at the Single Junction (K) of all SiPM cathode terminals.
Figure 3 illustrates a circuit design embodiment where the timing signal is obtained at the Single Junction (P_{zk}) of all SiPM cathodic terminals, but including the PCB routed delay lines in the schematic.
Figure 4 illustrates a circuit design embodiment where the timing signal is obtained at the Single Junction (A) of all SiPM anodic terminals.
Figure 5 illustrates a circuit design embodiment where the timing signal is obtained as the differential signal between the Single Junction (K) of all SiPM cathodic terminals and the Single Junction (P_{za}) of all SiPM anodic terminals indirectly wired.
Figure 6 illustrates a circuit design embodiment where the timing signal is obtained as the differential signal between the Single Junction (A) of all SiPM anodic terminals and the Single Junction (P_{zk}) of all SiPM cathodic terminals indirectly wired.
Figure 7 illustrates a circuit design embodiment where the timing signal is obtained at the Single Junction (P_{zk}) of all SiPM cathodic terminals indirectly wired.
Figure 8 illustrates a circuit design embodiment where the timing signal is obtained at the Single Junction (P_{za}) of all SiPM anode terminals indirectly wired.
Figure 9 shows a general diagram of the readout network topology according to the invention, similar to that of Figure 1, wherein the readout network is made up of the stacking of several lower-level readout networks arranged in the form of columns.
Figure 10 shows the diagram of the same topology seen in Figure 9, but by means of the stacking of lower level readout networks arranged in the form of rows.
Figure 11 shows a particularization of the more general scheme of figure 9, in which the CF filters are schematized as C-R networks in series, in the same way as in figures 2 to 8, with the exception that we maintain the names of each filter in the form CFᵢⱼ, as they are in Figure 9 from which they are derived.
Figure 12 shows the same particular scheme as figure 11, in which we apply a simplification of the sub-circuit of the CF filters in series, which allows us to reduce the number of components, leaving only one common capacitor for all the C-R filters. in series, and a resistor on each load output to the filter.
Figure 13 shows two TOF-PET detector diagrams, with a column low-level readout network topology, which includes SiPM detectors coupled to a scintillating crystal, the one on the left being continuous crystal and the one on the right being pixelated..
Figure 14 shows two TOF-PET detector diagrams, with a readout network topology stacked in columns, which includes the stacking of detectors like those in Figure 13, either made of continuous glass as the one on the top, or pixelated as the one on the bottom.

### Detailed description and examples

The basic cell of the present invention includes a repeatable pack of components and connections such as that labelled at Fig 1, as the SiPM the SiPM cathode K, the SiPM anode A, the Delay, the bias filters TBF and BBF, the output filter CF, the output signal X, and other components from specific embodiments related to other figures (Rf, Cf, Ce, Re, etc).

The cells in the present invention are arranged in a matrix of n elements, wherein n = p x q, being p: rows and q columns. We may call i, to an index to make reference to all cells, provided that ii ranges from 1 to n.

So, according to this convention, it is possible to make reference to any component of a Cell in the photodetector arrangement, using its own generic name followed by this index, such as SiPMᵢ, Kᵢ, Aᵢ, TBFᵢ, BBFᵢ, CFᵢ, etc.

In the readout network topology (Figure 1 Circuit design topology), all similar terminals of one of the two types of the photodetector terminals (either the anodic ones or the cathodic ones) are wired to a Single Junction at the PCB (for example at Pzk or Pza), wherein the Single Junction is further split into two routes for further connections.

On the one hand, a first route connects the Single Junction, by means of a specific bias impedance (Zₖ), to the proper source polarity (Vbias). The bias impedance is working as part of the photodetector bias circuit, and its resistance is set to be less than 10% compared to the resistance of the rest of the route from the Single Junction to GND, so that the voltage at the Single Junction is virtually the same as the voltage of the source which is feeding the other terminal of the impedance, preventing the individual photodetector capacitances to be added.

We are using here, by simplicity, the specific labeling of the Figure 1 Circuit design topology, which is set according to the specific voltage polarity setting (however, the polarity can be reversed). So, this polarity is Vbias, because the terminals wired to the Single Junction are the cathodes, otherwise it should be GND if the wired terminals were the anodes.

In the same way, filters are established in the polarization route from the photodetector both towards Vbias and towards GND and their parameters are established so that the filters can be canceled or activated according to the particular embodiment. In particular, in this invention we deal with configurations where the filters are set alternately, either on the polarization route between photodetector and Vbias (in which case we call it TBF) or they are set on the polarization route between photodetector and GND (in which case we call it BBF). In the different particular embodiments, only the filters that are actually active will be represented.

For the general case of this topology, the power connections: Vbias and GND are considered exchangeable provided that the components polarities are adjusted accordingly, so that different configurations are obtained on the same basis, representing different signal and power source polarities (positive, negative and differential), as we will see below in a set of different embodiments. Furthermore, giving specific limit values for some components, will turn the general circuit into a specific embodiment of the invention.

On the other hand, a second route feeds the unified signal created at the Single Junction, into a fast amplifier, obtaining a single timing signal regarding the complete photodetector arrangement, no matter which individual photodetector it comes from.

The bias impedance is labelled Zₐ when it is placed in the unified route from the photodetector anodes to Ground (GND), and it is labelled Zₖ when it is placed in the unified route from the photodetector cathodes to Vbias.

The Single Junction may appears near different source polarities, and connecting different photodetector terminals (for example, SiPM terminals) , according to the specific embodiment used, based on the described circuit topology. When the Single Junction is wiring anodic terminals coming directly from the photodetector arrangement it is simply labelled A, while it is labelled K, when the Single Junction is connecting the cathode terminals coming directly from the photodetector matrix (e.g. SiPM).

Moreover, in some embodiments there is some circuitry replicated between each photodetector terminal that we want to join, and the effective PCB point at the Single Junction, where we wire up all together, so that we have an indirect connection between the Single Junction and the photodetector arrangement terminals.

When the Single Junction is wiring connections that are coming indirectly from the photodetector arrangement anodic terminals it is labelled as P_{za}, while it is labelled as P_{zk}, when the Single Junction is wiring the cathode terminals coming indirectly from the photodetector (as for example SiPM) arrangement.

After these clarifications, it follows that, when we refer in general to the unified timing signal that is carried from the Single Junction point (or points) into the fast amplifier to obtain a single timing signal regarding the whole photodetector, no matter which individual photodetector it comes from, this also refers to all other equivalent points that work as the Single Junctions in the different embodiments, labelled as A, K, P_{za} and P_{zk}. According to the embodiments that we will describe below in detail, we may obtain single polarity timing signals from any of these labelled points or differential timing signals using valid combinations of these labelled points, as we will see below.

The routes to each individual photodetector are tuned for synchronization of the starting edges of any signal coming from any individual photodetector. These routes could be represented with delay lines (Delayᵢ), (i ranges from 1 to n, and n = p x q in a matrix) in series with each individual photodetector.

All the other similar photodetector terminals, that were not directly or indirectly wired to the Single Junction, are separately connected to two connection types: On the one hand, these are connected to the other power source polarity by means of a parallel set of individual filtering circuits (BBFᵢ), (bias filters) (and eventually including another bias impedance (either Zₐ or Zₖ) in series with the BBFᵢ filters, and on the other hand, these are extracted as independent output signals (Xᵢ) by means of a parallel set of individual filtering circuits (CFᵢ) (charge filters), that can be used to feed any readout network scheme to obtain X and Y coordinates, Depth-of-Interaction values and energy.

The combined adjustment of the charge filters CFᵢ and the bias filters BBFᵢ and TBFᵢ the bias impedances, Zₖ and Zₐ, the capacitances of the individual photodetectors and the routed delays (Delayᵢ), through each connection of the individual photodetectors, to obtain an optimal circuit response, allowing us to separately obtain information about the captured charge and the position of the individual photodetectors involved in the event, as well as a single, accurate, phase tuned timing signal, regarding the same event at any part of the whole photodetector, no matter which individual photodetector it came from.

Furthermore, a fast amplifier is electrically coupled to the parallel common output of the photodetector, where the single timing signal is generated. This amplifier is electrically coupled to this timing signal, is characterized by a fast response and a low input impedance, creating a low-impedance path for fast transition signals, which is going to improve (to reduce) the total parasitic capacitance of the parallel individual photodetectors arrangement, improving the time response of the whole photodetector. Also, the readout electronics of the invention creates a reduced number of positioning signals, from (p x q) signals to (p + q) signals.

The Single Junction is connected by means of a specific bias impedance, to the proper source polarity (Vbias). The bias impedance is working as part of the photodetector bias circuit, and its resistance is set to be less than 10% compared to the resistance of the rest of the route from the Single Junction to GND, so that the voltage at the Single Junction is virtually the same as the voltage of the source which is feeding the other terminal of the impedance, preventing the individual photodetector capacitances to be added. One has to be aware that the resistance is the real part of the complex numeric value of the impedance. In these conditions, the terminals of the whole photodetector are virtually connected between Vbias and GND, minimizing their mutual interferences, with a behavior similar to that if they were not in a parallel connection, so, their capacitances are nor added from the point of view of the Xᵢ signals.

On the other hand, from the point of view of the timing signal, the bias impedance together with the amplifier A_{f}, creates a high frequency band-pass filter that absorbs and amplifies any starting edge of a fast signal coming from any individual photodetector to provide a single common timing signal.

In the case of the parallel set of charge filters, these are trimmed to obtain at each one of the different Xᵢ outputs, a bandwidth apart enough from that of the timing signal, but also at a lower frequency, in order to be able to obtain an accurate measurement of the charges involved in the measured event, without regarding the time consumed to accomplish this. These outputs represent individual uncoupled signals from each individual photodetector, that can be fed to other suitable readout networks, or different electronic circuits, that collect all the charges of the photodetector, according to the sensing scheme used to obtain values representing magnitudes such as planar X & Y spatial coordinates, the event energy and the Depth-of-Interaction.

In the case of the bias filters BBFᵢ, TBFᵢ those are trimmed, together with the bias impedance, to match individually the bias current of each individual photodetector and to obtain at the Single Junction, a very high and narrow frequency response, close to the highest frequency components of the signal coming from the photodetector, in order to generate an accurate and early time response output, without affecting the belated signals (Xᵢ), which contain charge information and will appear later. The belated signal is a high frequency signal too, but having lower bandwidth than the time-response signal, allowing us to separate both by frequency filtering.

As a result, a very fast output signal will appear at the Single Junction, during a very short time frame as a response to any high-speed transition signal coming from any of the individual photodetectors (such as SiPM).

The resulting single timing signal is injected to the mentioned fast amplifier, which is designed to operate in the highest frequency band of the photodetector response, to generate a single, time-accurate and early time response, without affecting the belated signal with charge information, directed by separate paths, following each of the other SiPM terminals.

### Example 1 and 2

In a specific embodiment of the design (Figure 2), all the SiPMᵢ detectors (SiPM₁ to SiPMₙ) are connected in such a way that, all the cathodic terminals (Kᵢ) are wired together to obtain the Single Junction K, which is then connected to the bias source by means of a single impedance (Zₖ), while all the anodic terminals (Aᵢ) are separately connected to two connection types: On the one hand, these are separately connected to GND by means of a parallel set of individual filtering circuits (Cfᵢ∥Rfᵢ) working as the BBFᵢ filters at Figure 1. On the other hand, these are extracted as independent output signals (Xᵢ) by means of a parallel set of individual filtering circuits (Ceᵢ-Reᵢ) working as the Cfᵢ filters of Figure 1. In this case, the impedance Zₐ of the Figure 1 is 0, thus shortened to GND, and the TBFᵢ filters are cancelled by shortening the filter resistance.

The mentioned filtering circuits are tuned in such a way that for high-speed transition signals, all the individual photodetectors virtually share the same connection points (parallel connection between K and GND) allowing the generation of a very fast signal S_{K} at the unified terminal K, representing the time response of the whole photodetector, no matter which individual photodetector the signal comes from. The signal S_{K} is used to feed a fast amplifier (A_{f}), designed to operate specifically at the highest frequency band of the individual photodetectors response, in order to generate an accurate and early time response output, without affecting the belated signals (Xᵢ), which contain charge information and will appear later following each of the other photodetector terminals Aᵢ, in the other route, through the parallel set of individual filtering circuits (Ceᵢ-Reᵢ).

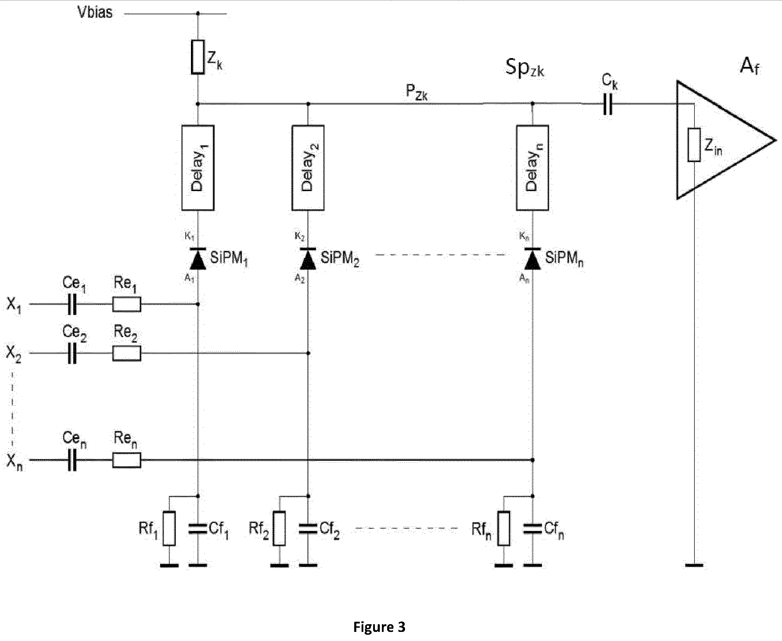

All individual photodetectors printed circuit board (PCB) routing connections are "time delay tuned" in a way that the starting edges of any signal coming from any individual photodetector will arrive synchronized to the circuit point where the single common timing signal is obtained, (K, for this case). This could be represented as a delay line inserted in series connection to each individual photodetector, so that the circuit of Figure 2 becomes Figure 3. All the schematics hereafter are considered to include the PCB routed delay lines in series with each individual photodetector, although they will not be drawn in the schematics for simplicity.

### Example 3

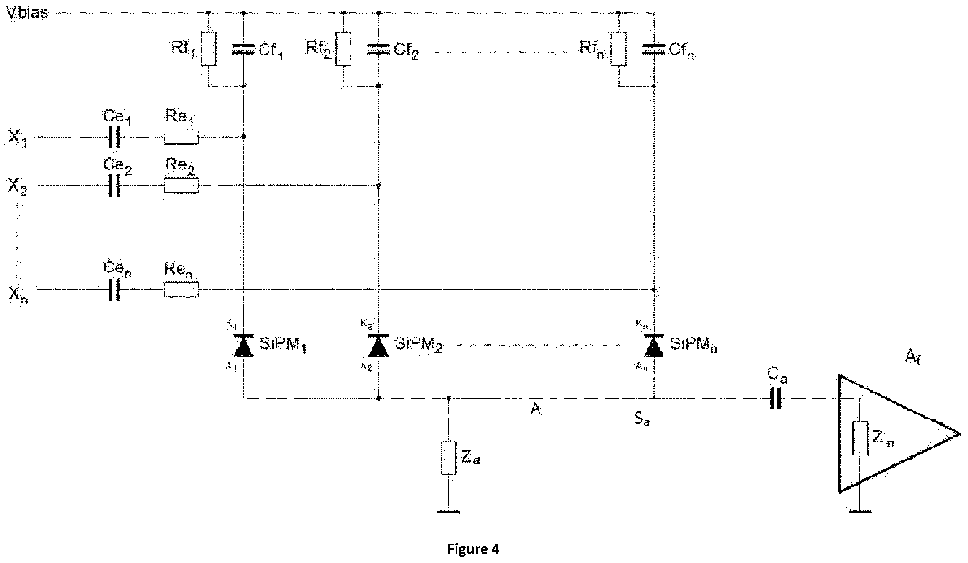

In a second specific embodiment of the design (Figure 4]), all the SiPMᵢ photodetectors (SiPM₁ to SiPMₙ) are connected in such a way that, all the anodic terminals (Aᵢ) (i ranges from 1 to n) are wired together to obtain the Single Junction A, which is then connected to GND by means of a single impedance (Zₐ), while all the cathodic terminals (Kᵢ) are separately connected to two connection types: On the one hand, these are separately connected to the bias power source by means of a parallel set of individual filtering circuits (Cfᵢ∥Rfᵢ), working as the TBFi filters at the Figure 1. On the other hand, these are extracted as independent output signals (Xᵢ) by means of a parallel set of individual filtering circuits (Ceᵢ-Reᵢ).

In this case, the impedance Zk of the Figure 1 is 0, thus shortened to Vbias, and the BBFᵢ filters are cancelled by shortening the filter resistance.

The mentioned filtering circuits are tuned in such a way that for high-speed transition signals, all the individual photodetectors virtually share the same connection points (parallel connection between A and Vbias), allowing the generation of a very fast signal Sₐ at the unified terminal A representing the time response of the whole photodetector, no matter which individual photodetector the signal comes from. The signal Sₐ is used to feed a fast amplifier A_{f}, designed to operate specifically at the highest frequency band of the photodetector response, in order to generate an accurate and early time response output, without affecting the belated signals (Xi) which contain charge information, and will appear later, following each of the other individual photodetector terminals Kᵢ, in the other route, through the parallel set of individual filtering circuits (Ceᵢ-Reᵢ).

### Example 4

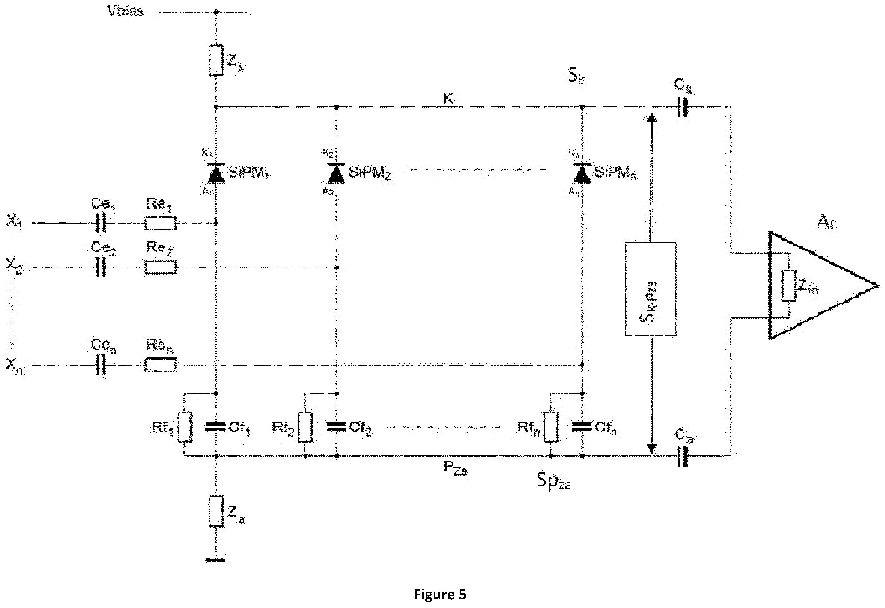

In a third specific embodiment of the design (Figure 5]), all the individual photodetectors (SiPM₁ to SiPMₙ) are connected in such a way that, all the cathodic terminals (Kᵢ) (i ranges from 1 to n) are wired together to obtain the Single Junction K, which is further connected to the bias source by means of a single impedance (Zₖ), while all the anodic terminals (Aᵢ) are separately connected to two connection types: On the one hand, these are separately connected to a Single Junction (Pzₐ) by means of a parallel set of individual filtering circuits (Cfᵢ∥Rfᵢ) working as the BBFᵢ filters of the Figure 1, and then connected from here to GND by means of a single impedance Zₐ. On the other hand, these are extracted as independent output signals (Xᵢ) by means of a parallel set of individual filtering circuits (Ceᵢ-Reᵢ). In this case the TBFᵢ filters are cancelled by shortening the filter resistance.

The mentioned filtering circuits are tuned in such a way that for high-speed transition signals, all the individual photodetectors virtually share the same connection points (parallel connection between K and Pzₐ), allowing the generation of a differential very fast signal S_{K-PZA} between the unified terminal K, and the unified terminal P_{ZA} representing the time response of the whole photodetector, no matter which photodetector the signal comes from. The signal S_{K-Pza} is used to feed differentially a fast amplifier (A_{f}), designed to operate specifically at the highest frequency band of the individual photodetectors response bandwidth, in order to generate an accurate and early time response output, without affecting the belated signals (Xi) which contain charge information, and will appear later, following each of the other photodetector terminals Aᵢ.

### Example 5

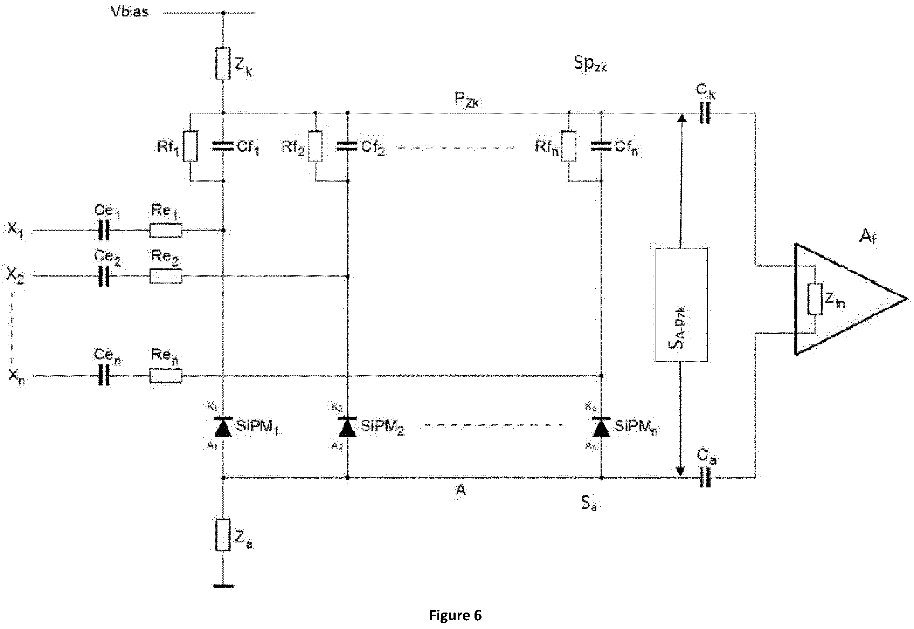

In a fourth specific embodiment of the design (Figure 6), all the individual photodetectors (SiPM₁ to SiPMₙ) are connected in such a way that, all the anodic terminals (Aᵢ) (i ranges from 1 to n) are wired together to obtain the Single Junction A, which is further connected to GND by means of a single impedance (Zₐ), while all the cathodic terminals (Kᵢ) are separately connected to two connection types: On the one hand, these are separately connected to a Single Junction (P_{zk}) by means of a parallel set of individual filtering circuits (Cfi_{∥}Rfᵢ) working as the TBFᵢ filters at the Figure 1, and then connected from here to Vbias by means of a single impedance Zₖ. On the other hand, these are extracted as independent output signals (Xᵢ) by means of a parallel set of individual filtering circuits (Ceᵢ-Reᵢ). In this case the BBFᵢ filters are cancelled by shortening the filter resistance.

The mentioned filtering circuits are tuned in such a way that for high-speed transition signals, all the individual photodetectors virtually share the same connection points (parallel connection between A and P_{Zk}), allowing the generation of a differential very fast signal S_{A-Pzk} between the unified terminal A, and the unified terminal P_{Zk} representing the time response of the whole photodetector, no matter which individual photodetector the signal comes from. The signal S_{A-Pzk} is used to feed differentially a fast amplifier (A_{f}), designed to operate specifically at the highest frequency band of the detector response, in order to generate an accurate and early time response output, without affecting the belated signals (Xi) which contain charge information, and will appear later, following each of the other photodetector terminals Kᵢ.

### Example 6

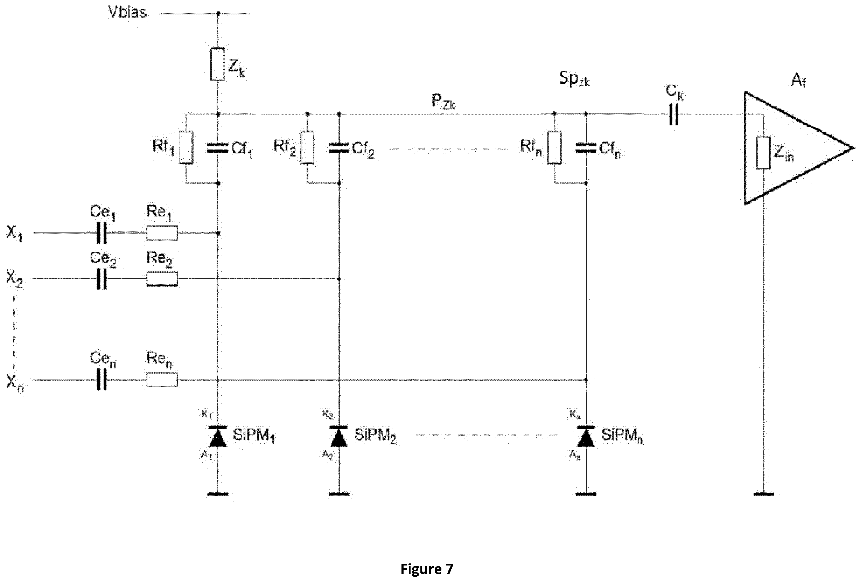

In a fifth specific embodiment of the design (Figure 7), all the individual photodetectors (SiPM₁ to SiPMₙ) are connected in such a way that, all the anodic terminals (Aᵢ) (i ranges from 1 to n) are wired to GND, while all the cathodic terminals (Kᵢ) are separately connected to two connection types: On the one hand, these are separately connected to a Single Junction (P_{zk}) by means of a parallel set of individual filtering circuits (Cfᵢ∥Rfᵢ) working as the TBFᵢ filters of the Figure 1, and further connected from here to Vbias by means of a single impedance Zₖ. On the other hand, these are extracted as independent output signals (Xᵢ) by means of a parallel set of individual filtering circuits (Ceᵢ-Reᵢ). In this case, the impedance Za of the Figure 1 is 0, thus shortened to Vbias, and the BBFᵢ filters are cancelled by shortening the filter resistance.

The mentioned filtering circuits are tuned in such a way that for high-speed transition signals, all the individual photodetectors virtually share the same connection points (parallel connection between P_{Zk} and GND), allowing the generation of a very fast signal S_{Pzk} at the unified terminal P_{Zk} representing the time response of the whole photodetector, no matter which photodetector the signal comes from. The signal S_{Pzk} is used to feed a fast amplifier (A_{f}), designed to operate specifically at the highest frequency band of the detector response, in order to generate an accurate and early time response output, without affecting the belated signals (Xi) which contain charge information, and will appear later, following each of the photodetector terminals Kᵢ, in the other route, through the parallel set of individual filtering circuits (Ceᵢ-Reᵢ).

### Example 7

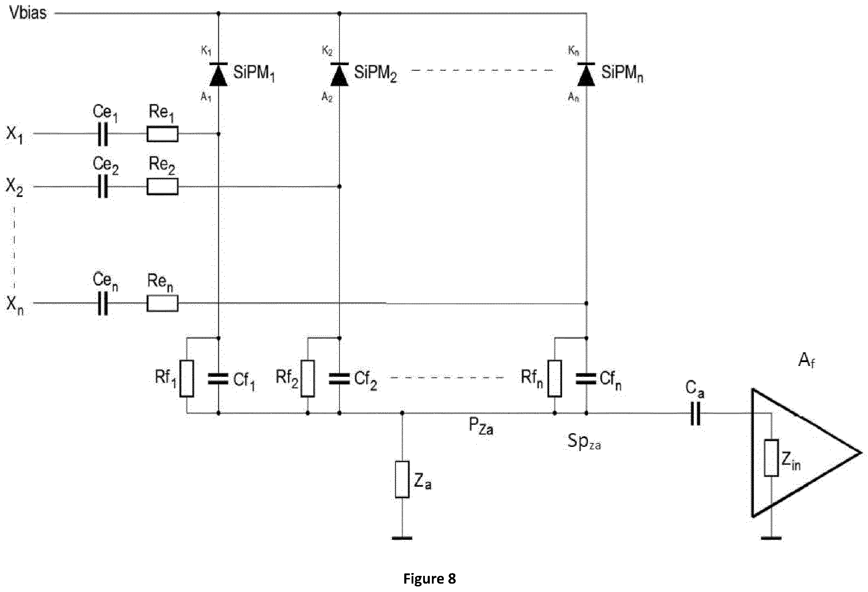

In a sixth specific embodiment of the design (Figure 8), all the individual photodetectors (SiPM₁ to SiPMₙ) are connected in such a way that, all the cathodic terminals (Kᵢ) (i ranges from 1 to n) are wired to Vbias, while all the anodic terminals (Aᵢ) are separately connected to two connection types: On the one hand, these are separately connected to a Single Junction (Pzₐ) by means of a parallel set of individual filtering circuits (Cfᵢ∥Rfᵢ) working as the BBFᵢ filters of the Figure 1, and further connected from here to GND by means of a single impedance Zₐ. On the other hand, these are extracted as independent output signals (Xᵢ) by means of a parallel set of individual filtering circuits (Ceᵢ-Reᵢ). In this case, the impedance Zk of the Figure 1 is 0, thus shortened to Vbias, and the TBFi filters are cancelled by shortening the filter resistance.

The mentioned filtering circuits are tuned in such a way that for high-speed transition signals, all the individual photodetectors virtually share the same connection points (parallel connection between Vbias and Pzₐ), allowing the generation of a very fast signal S_{Pza} at the unified terminal P_{Za} representing the time response of the whole photodetector, no matter which individual photodetector the signal comes from. The signal S_{Pza} is used to feed a fast amplifier (A_{f}), designed to operate specifically at the highest frequency band of the detector response, in order to generate an accurate and early time response output, without affecting the belated signals (Xᵢ) which contain charge information, and will appear later, following each of the photodetector terminals Aᵢ, in the other route, through the parallel set of individual filtering circuits (Ceᵢ-Reᵢ).

### Example 8

In a seventh design embodiment (Figure 9), we use the stacking capacity of this read network topology, described in the second object of the invention, to show a package of 6 design variants, equivalent to the 6 embodiments described in the 7 previous examples. The difference consists in using several lower-level readout networks, stacked to form a higher-level readout network, either by stacking columns (Figure 9) or by stacking rows (Figure 10). In all cases, both the constituent lower-level networks and the obtained higher-level networks are part of the readout network topology disclosed here.

Any of the variants of readout networks, such as the 6 already described in the previous examples, can serve as a lower level readout network (or building block) from which the stacked readout networks of example 8 are obtained. There is also a set of filter variants that can be placed in place of the blocks called TBF, BBF, and CF, beyond the 6 particular schemes shown in Figures 3 to 8. From this arises a wide combinatorics of particular embodiments, all of which maintain the topology described. Representative variants of the readout network topology are shown.

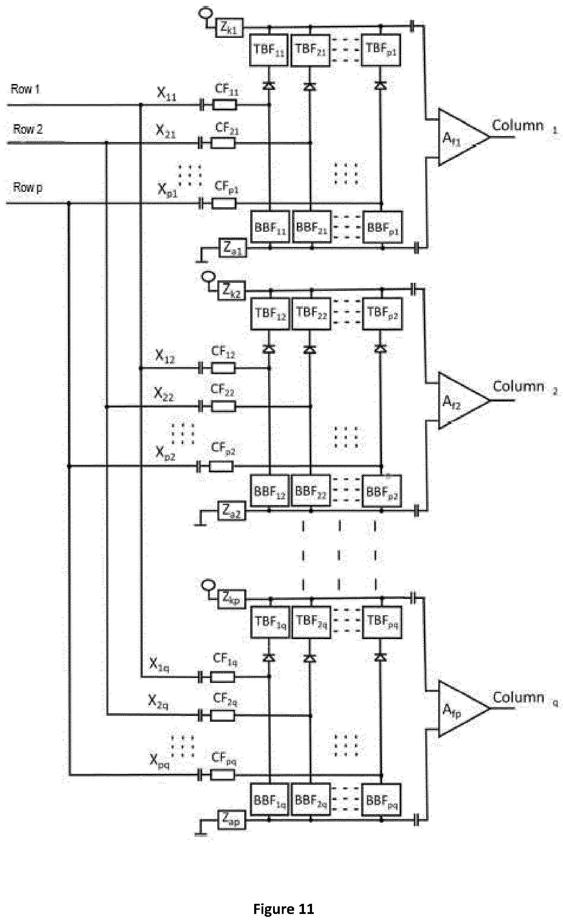

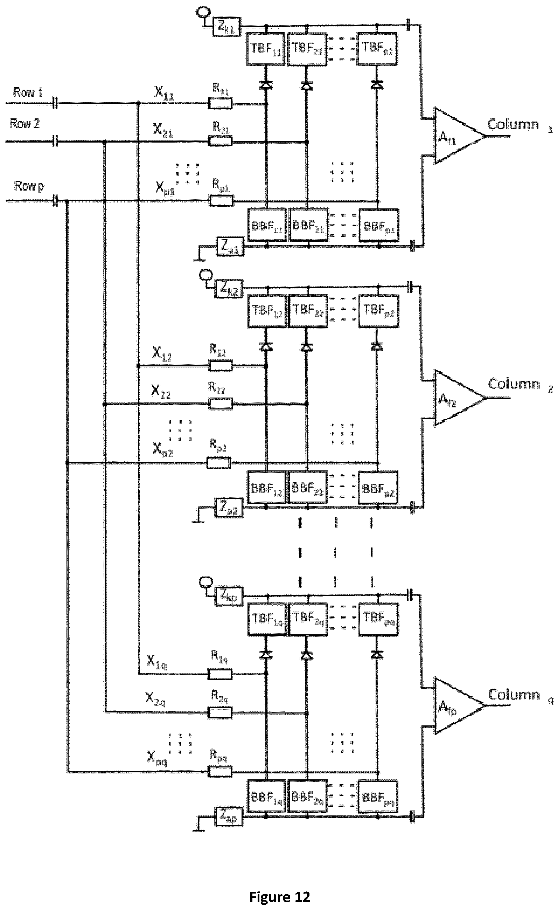

The readout network circuits of Figure 11 or Figure 12 are the result of the stacking of readout networks of a column and can have any of the networks described in the 7 previous embodiments as a lower level readout network. We can refer to any of these examples to know the specific details of each one. They could have, for example, 16 readout networks, each one having one column and 16 rows (p=16). This allows us to form a stacked readout network of 16 rows + 16 columns. These 32 signs can be simultaneously read to know the impact positions anywhere in the detector, and an impact timestamp of each column.

The circuit in Figure 12 is derived from that in Figure 11, as a simplification, since all the C-R filters are linked in series at one of the terminals, that part of the circuit can be replaced by an equal number of equivalent filters created at starting from a common component (for example the capacitor C), after which the other components (for example all the resistors of the replaced filters) are derived by separate connections.

## Claims

1. A readout network topology for positron emission tomography, PET, with time of flight -TOF- capabilities to extract position and time information in the area of gamma-ray detection comprising:
- a plurality of individual photodetectors arranged in p x q matrix configuration on an electronic Printed Circuit Board - PCB- to conform a photodetector capable to process the light arriving to the matrix area and providing information to determine spatial coordinates and timing signal, each individual photodetector having an anodic terminal and a cathodic terminal,
- a connection of all similar terminals of one of the two types of the photodetector terminals, either the anodic ones or the cathodic ones, to a Single Junction at the PCB, which further splits into two routes,
- the first one of these routes connects the Single Junction, by means of a specific impedance, to the proper source polarity, wherein "proper polarity" means: Vbias if the wired terminals to the Single Junction are the cathodes or ground, GND, if the wired terminals to the Single Junction are the anodes, wherein the specific impedance, either Za if the wired terminals are the anodes, or Zk if the wired terminals are the cathodes, is working as part of the photodetector bias circuit, and its resistance is set to be less than 10% compared to the resistance of the rest of the route from the Single Junction to GND and,
- the second one of these routes feeds the unified signal created at the Single Junction, into a fast amplifier, in order to obtain a single timing signal regarding the whole photodetector, no matter which specific individual photodetector it comes from
- an individual connection of all the photodetector terminals, different from those ones connected to the Single Junction, by means of two separate filtering circuits for each photodetector terminal,
- the first filtering circuits named bias filters (TBF, BBF), complete the bias circuits for all individual photodetectors, connecting, by means of them, the individual photodetector terminals to the proper source polarity, wherein "proper" means that it is Vbias if the photodetector terminal is the cathode or GND if the photodetector terminal is the anode and,
- the second filtering circuit named charge filters function as output signal filters, to obtain individual current signals from each individual photodetector, representing the quantity of light captured by each individual photodetector in the arrangement, giving rise to uncoupled Xᵢ signals.

2. A readout network topology according to claim 1, wherein the timing signal is selected as one of the following:
- the sum at the Single Junction (K) of all the individual cathode terminals,
-- the sum at the Single Junction (Pzk) of all individual photodetectors cathode terminals including the routed delay lines,
- the sum, at the Single Junction (A), of all anode terminals of the individual photodetectors.
- the sum at the Single Junction (Pzk) of all individual photodetectors cathode terminals indirectly wired.
- the sum at the single junction (A) of all individual photodetectors anode terminals indirectly wired.

3. A readout network topology according to claim 1, wherein the timing signal is selected as:
- the result of the differential signal between the Single Junction (K) of all the cathode terminals of the individual photodetectors and the Single Junction (Pza) of all anode terminals of the individual photodetectors indirectly connected or
- the result of the differential signal between the Single Junction (A) of all individual photodetectors anodic terminals and the Single Junction (Pzk) of all individual photodetectors cathode terminals indirectly wired.

4. A readout network topology according to any of the preceding claims, wherein the individual photodetectors are SiPM.

5. A higher-level readout network topology for positron emission tomography, PET, comprising a plurality of lower-level readout networks, comprised in the readout network topology defined in any of claims 1 to 4.

6. A higher level readout network topology according to claim 5, with p rows and q columns comprising:
a number q of lower level readout networks, each having a single column and a number p of rows, or
- a number p of lower level readout networks, each one having a single row and a number of columns q.

7. A nuclear detector module comprising one or more readout network topologies as the ones defined in any of claims 1 to 6.

8. A nuclear detector module according to claim 7, wherein there are a plurality of readout networks arranged, that are selected as:
- one column, and a p number of rows, or
- one row, and a number q of columns,
in such a way that the different time reference signals obtained from each readout network are connected to a load reading scheme to obtain energy and position information with respect to the plurality of readout networks.

9. A nuclear detector module according to claim 8, consisting of a number q of readout networks arranged in columns, each one of which has a column, and a number p of rows, such that the different q time reference signals obtained from each readout network are connected to a load reading scheme to obtain energy and position information with respect to the columns of the plurality of readout networks, while the different p load signals Xi are connected to a reading scheme of loads to obtain energy and position information with respect to the rows of the plurality of readout networks.

10. A nuclear detector module according to claim 8, consisting of a number p of readout networks arranged in rows, each one of which has one row, and a number q of columns, such that the different p time reference signals obtained from each readout network are connected to a load reading scheme to obtain energy and position information with respect to the rows of the plurality of readout networks, while the different q load signals Xi are connected to a reading scheme of loads to obtain energy and position information with respect to the columns of the plurality of readout networks.

11. A nuclear detector module according to any one of claims 7, 8, 9 or 10, further comprising:
- a scintillator crystal part facing the photodetector.
- a housing to protect the module from external light and mechanical stress,
- an analog to digital conversion electronics capable of processing all positioning and energy signals obtained, and transmitting these through a high-speed computer interface to be processed on the fly, or stored in a computer disk, to be later processed, as well as preserving the timing signal.

12. A ToF-PET System comprising
- a plurality of detector modules as the ones described in any one of claims 7 to 11, arranged in a mechanical gantry, having a symmetrical configuration, with all the modules surrounding and facing the volume of detection that can be covered by them,
- a coincidence processing electronic module capable to process the timing signals coming from a plurality of modules as the ones described in claim 11 to provide proper matching among multiple combinations of modules, allowing to effectively process as a single block, all signals related to any single PET event, no matter which module they come from, as well as capable to digitize and transmit all the timing related signals through a high-speed computer interface to be processed on the fly, or stored in a computer disk, to be later processed, including time of flight information of all detected events.

13. The ToF-PET system according to claim 12, further comprising a software module capable of processing all the data stored in the computer or received in real time, to provide high-quality ToF-PET images, using any computer or digital interface.

14. A method for obtaining time and position information from the signals coming from a ToF-PET photodetector, in a detector module of a PET device, the method comprising
- arranging a scintillation crystal in a detector module
- arranging a readout network topology as the one defined in any of claims 1 to 12,
- entering a gamma ray into the module and impinging the scintillator crystal volume, creating a light beam which spreads out everywhere, including the readout network photodetector area,
- entering the light beam coming from a scintillator crystal into the readout network photodetector area,
- converting the light beam into a spreaded distribution of electrical signals coming from the whole photodetector area,
- bringing the first signal detected from any part of the photodetector to a fast amplifier to point it out as the output timing signal, representing the exact instant of the arrival of the gamma ray to the scintillator crystal volume,
- bringing every one of the electrical signals coming from the whole photodetector area, by means of charge filters to provide uncoupled charge signal outputs compatible with readout electronic circuits commonly used to extract position and energy of the gamma ray.

15. A method according to claim 14 that further comprises handling data from at least two modules, providing information according to claim 14 obtaining the ToF lines of response required to create a ToF-PET image.

## Patentansprüche

1. Auslesenetzwerktopologie für Positronenemissionstomografie, PET, mit Fähigkeiten der Flugzeit -TOF- zur Gewinnung von Positions- und Zeitinformationen im Bereich der Gammastrahlendetektion, umfassend:
- eine Vielzahl von einzelnen Fotodetektoren, die in einer p × q-Matrixkonfiguration auf einer elektronischen Leiterplatte -PCB- angeordnet sind, um einen Fotodetektor aufzubauen, der in der Lage ist, das auf den Matrixbereich auftreffende Licht zu verarbeiten und Informationen zum Bestimmen von Raumkoordinaten und einem Zeitgebersignal bereitzustellen, wobei jeder einzelne Fotodetektor eine Anodenklemme und eine Kathodenklemme aufweist,
- eine Verbindung aller gleichartigen Klemmen eines der beiden Typen der Fotodetektorklemmen, entweder der anodischen oder der kathodischen, mit einem **E**inzelanschluss auf der PCB, der sich wiederum in zwei Wege aufteilt,
- wobei der erste dieser Wege den Einzelanschluss mittels einer spezifischen Impedanz mit der richtigen Quellenpolarität verbindet, wobei "richtige Polarität" Folgendes bedeutet: Vbias, falls die drahtgebundenen Klemmen zum Einzelanschluss die Kathoden oder Masse, GND, sind, falls die an den Einzelanschluss angeschlossenen Klemmen die Anoden sind, wobei die spezifische Impedanz, entweder Za, falls die drahtgebundenen Klemmen die Anoden sind, oder Zk, falls die drahtgebundenen Klemmen die Kathoden sind, als Teil der Vorspannungsschaltung des Fotodetektors fungiert und ihr Widerstand so eingestellt ist, dass er weniger als 10 % des Widerstands des Restes des Weges vom Einzelanschluss zu GND beträgt, und
- wobei der zweite dieser Wege das an dem Einzelanschluss erzeugte vereinheitlichte Signal in einen schnellen Verstärker versorgt, um ein einziges Zeitgebersignal bezüglich des gesamten Fotodetektors zu erhalten, unabhängig davon, von welchem einzelnen Fotodetektor es stammt
- eine einzelne Verbindung aller Fotodetektorklemmen, die sich von den an den Einzelanschluss verbundenen unterscheiden, mittels zweier separater Filterschaltungen für jede Fotodetektorklemme,
- wobei die ersten Filterschaltungen, die als Vorspannungsfilter (TBF, BBF) bezeichnet werden, die Vorspannungsschaltungen für alle einzelnen Fotodetektoren vervollständigen, wobei sie die einzelnen Fotodetektorklemmen mit der richtigen Quellenpolarität verbinden, wobei "richtig" bedeutet, dass es Vbias ist, falls die Fotodetektorklemme die Kathode ist, oder GND, falls die Fotodetektorklemme die Anode ist, und
- die zweite Filterschaltung, die als Ladungsfilter bezeichnet wird, als Ausgangssignalfilter fungiert, um von jedem einzelnen Fotodetektor einzelne Stromsignale zu erhalten, die die von jedem einzelnen Fotodetektor in der Anordnung erfasste Lichtmenge darstellen und zu entkoppelten Xᵢ-Signalen führen.

2. Auslesenetzwerktopologie nach Anspruch 1, wobei das Zeitgebersignal als eines der folgenden ausgewählt wird:
- die Summe aller einzelnen Kathodenklemmen am Einzelanschluss (K),
-- die Summe aller einzelnen Kathodenklemmen der Fotodetektoren am Einzelanschluss (Pzk), einschließlich der verlegten Verzögerungsleitungen,
- die Summe aller Anodenklemmen der einzelnen Fotodetektoren am Einzelanschluss (A),
- die Summe aller indirekt drahtgebundenen Kathodenklemmen der einzelnen Fotodetektoren am Einzelanschluss (Pzk),
- die Summe aller indirekt drahtgebundenen Anodenklemmen der einzelnen Fotodetektoren am Einzelanschluss (A).

3. Auslesenetzwerktopologie nach Anspruch 1, wobei das Zeitgebersignal als Folgendes ausgewählt wird:
- das Ergebnis des Differenzsignals zwischen dem Einzelanschluss (K) aller Kathodenklemmen der einzelnen Fotodetektoren und dem Einzelanschluss (Pza) aller Anodenklemmen der einzelnen Fotodetektoren, die indirekt verbunden sind, oder
- das Ergebnis des Differenzsignals zwischen dem Einzelanschluss (A) aller Anodenklemmen der einzelnen Fotodetektoren und dem Einzelanschluss (Pzk) aller Kathodenklemmen der einzelnen Fotodetektoren, die indirekt drahtgebunden sind.

4. Auslesenetzwerktopologie nach einem der vorhergehenden Ansprüche, wobei die einzelnen Fotodetektoren SiPMs sind.

5. Auslesenetzwerktopologie höherer Ebene für Positronenemissionstomografie, PET, die eine Vielzahl von Auslesenetzwerkken niedrigerer Ebene umfasst, die in der in einem der Ansprüche 1 bis 4 definierten Auslesenetzwerktopologie enthalten sind.

6. Auslesenetzwerktopologie höherer Ebene nach Anspruch 5 mit p Zeilen und q Spalten, umfassend:
eine Anzahl q von Auslesenetzwerkken niedrigerer Ebene, von denen jedes eine einzelne Spalte und eine Anzahl p von Zeilen aufweist, oder
- eine Anzahl p von Auslesenetzwerkken niedrigerer Ebene, von denen jedes eine einzelne Zeile und eine Anzahl q von Spalten aufweist.

7. Nukleardetektormodul, umfassend eine oder mehrere Auslesenetzwerktopologien, wie sie in einem der Ansprüche 1 bis 6 definiert sind.

8. Nukleardetektormodul nach Anspruch 7, wobei eine Vielzahl von Auslesenetzwerkken angeordnet ist, die wie folgt ausgewählt sind:
- eine Spalte und eine Anzahl p von Zeilen oder
- eine Zeile und eine Anzahl q von Spalten,
sodass die unterschiedlichen Zeitreferenzsignale, die von jedem Auslesenetzwerkk erhalten werden, mit einem Lastausleseschema verbunden sind, um Energie- und Positionsinformationen in Bezug auf die Vielzahl von Auslesenetzwerkken zu erhalten.

9. Nukleardetektormodul nach Anspruch 8, bestehend aus einer Anzahl q von in Spalten angeordneten Auslesenetzwerkken, von denen jedes eine Spalte und eine Anzahl p von Zeilen aufweist, sodass die unterschiedlichen q Zeitreferenzsignale, die von jedem Auslesenetzwerkk erhalten werden, mit einem Lastausleseschema verbunden sind, um Energie- und Positionsinformationen in Bezug auf die Spalten der Vielzahl von Auslesenetzwerkken zu erhalten, während die unterschiedlichen p Lastsignale Xi mit einem Ausleseschema von Lasten verbunden sind, um Energie- und Positionsinformationen in Bezug auf die Zeilen der Vielzahl von Auslesenetzwerkken zu erhalten.

10. Nukleardetektormodul nach Anspruch 8, bestehend aus einer Anzahl p von in Zeilen angeordneten Auslesenetzwerkken, von denen jedes eine Zeile aufweist, und einer Anzahl q von Spalten, sodass die unterschiedlichen p Zeitreferenzsignale, die von jedem Auslesenetzwerkk erhalten werden, mit einem Lastausleseschema verbunden sind, um Energie- und Positionsinformationen in Bezug auf die Zeilen der Vielzahl von Auslesenetzwerkken zu erhalten, während die unterschiedlichen q Lastsignale Xi mit einem Ausleseschema von Lasten verbunden sind, um Energie- und Positionsinformationen in Bezug auf die Spalten der Vielzahl von Auslesenetzwerkken zu erhalten.

11. Nukleardetektormodul nach einem der Ansprüche 7, 8, 9 oder 10, ferner umfassend:
- einen dem Fotodetektor zugewandten Szintillatorkristallteil,
- ein Gehäuse zum Schutz des Moduls vor externem Licht und mechanischer Beanspruchung,
- eine Analog-Digital-Wandlerelektronik, die in der Lage ist, alle erhaltenen Positions- und Energiesignale zu verarbeiten und diese über eine Hochgeschwindigkeits-Computerschnittstelle zur sofortigen Verarbeitung oder zur Speicherung auf einer Computerfestplatte zur späteren Verarbeitung zu übertragen sowie das Zeitgebersignal zu bewahren.

12. ToF-PET-System, umfassend
- eine Vielzahl von Detektormodulen, wie sie in einem der Ansprüche 7 bis 11 beschrieben sind, die in einem mechanischen Portal angeordnet sind, eine symmetrische Anordnung aufweisen und wobei alle Module das von ihnen abdeckbare Detektionsvolumen umgeben und diesem zugewandt sind,
- ein elektronisches Koinzidenzverarbeitungsmodul, das in der Lage ist, die von einer Vielzahl von Modulen, wie sie in Anspruch 11 beschrieben sind, stammenden Zeitgebersignale zu verarbeiten, um eine richtige Übereinstimmung zwischen mehreren Modulkombinationen herzustellen, wobei es möglich ist, alle Signale, die sich auf ein einzelnes PET-Ereignis beziehen, unabhängig davon, aus welchem Modul sie stammen, effektiv als einen einzigen Block zu verarbeiten, und dabei alle zeitgeberbezogenen Signale über eine Hochgeschwindigkeits-Computerschnittstelle zu digitalisieren und zu übertragen, um sie sofort zu verarbeiten oder auf einer Computerfestplatte zu speichern und später weiterzuverarbeiten, einschließlich der Flugzeitinformationen aller detektierten Ereignisse.

13. ToF-PET-System nach Anspruch 12, ferner umfassend ein Softwaremodul, das in der Lage ist, alle im Computer gespeicherten oder in Echtzeit empfangenen Daten zu verarbeiten, um hochwertige ToF-PET-Bilder unter Verwendung eines beliebigen Computers oder einer beliebigen digitalen Schnittstelle bereitzustellen.

14. Verfahren zum Erhalten von Zeit- und Positionsinformationen aus den von einem ToF-PET-Fotodetektor stammenden Signalen in einem Detektormodul einer PET-Vorrichtung, wobei das Verfahren Folgendes umfasst:
- Anordnen eines Szintillationskristalls in einem Detektormodul
- Anordnen einer Auslesenetzwerktopologie, wie sie in einem der Ansprüche 1 bis 12 definiert ist,
- Einleiten eines Gammastrahls in das Modul und Auftreffen auf das Volumen des Szintillatorkristalls, wobei ein Lichtstrahl erzeugt wird, der sich überall ausbreitet, einschließlich des Fotodetektorbereichs des Auslesenetzwerkks,
- Einleiten des von einem Szintillatorkristall kommenden Lichtstrahls in den Fotodetektorbereich des Auslesenetzwerkks,
- Umwandeln des Lichtstrahls in eine gestreute Verteilung elektrischer Signale, die aus dem gesamten Fotodetektorbereich stammen,
- Weiterleiten des ersten von einem beliebigen Teil des Fotodetektors detektierten Signals an einen schnellen Verstärker, um es als das Ausgangszeitgebersignal auszugeben, das den genauen Zeitpunkt des Eintreffens des Gammastrahls im Volumen des Szintillatorkristalls darstellt,
- Weiterleiten jedes einzelnen der aus dem gesamten Fotodetektorbereich stammenden elektrischen Signale mittels Ladungsfiltern, um entkoppelte Ladungssignalausgänge bereitzustellen, die mit Ausleseelektronikschaltungen kompatibel sind, die üblicherweise zur Ermittlung von Position und Energie des Gammastrahls verwendet werden.

15. Verfahren nach Anspruch 14, das ferner das Bearbeiten von Daten aus mindestens zwei Modulen, das Bereitstellen von Informationen nach Anspruch 14 und das Erhalten der zur Erzeugung eines ToF-PET-Bildes erforderlichen ToF-Antwortlinien umfasst.

## Revendications

1. Topologie de réseau de lecture pour la tomographie par émission de positrons, PET, avec des capacités de temps de vol -TOF- pour extraire des informations de position et de temps dans la zone de détection des rayons gamma, comprenant :
- une pluralité de photodétecteurs individuels agencés selon une configuration matricielle p x q sur une carte de circuit imprimé électronique -PCB- pour former un photodétecteur capable de traiter la lumière arrivant dans la zone matricielle et de fournir des informations pour déterminer les coordonnées spatiales et le signal temporel, chaque photodétecteur individuel ayant une borne anodique et une borne cathodique,
- une liaison de toutes les bornes similaires de l'un des deux types de bornes du photodétecteur
soit les bornes anodiques, soit les bornes cathodiques, à une **J**onction **U**nique sur la PCB, qui se divise ensuite en deux routes,
- la première de ces routes relie la Jonction unique, par l'intermédiaire d'une impédance spécifique, à la polarité de source appropriée, dans laquelle « polarité appropriée » signifie : Vbias si les bornes câblées à la Jonction Unique sont les cathodes ou la masse, GND, si les bornes câblées à la Jonction Unique sont les anodes, dans laquelle l'impédance spécifique, soit Za si les bornes câblées sont les anodes, soit Zk si les bornes câblées sont les cathodes, fonctionne comme faisant partie du circuit de polarisation de photodétecteur, et sa résistance est réglée pour être inférieure à 10 % par rapport à la résistance du reste de la route de la Jonction Unique à la masse, GND, et,
- la seconde de ces routes transfert le signal unifié créé au niveau de la Jonction Unique vers un amplificateur rapide, afin d'obtenir un signal temporel unique concernant l'ensemble du photodétecteur, quel que soit le photodétecteur individuel spécifique dont il provient
- une liaison individuelle de toutes les bornes de photodétecteur, différentes de celles liées à la Jonction Unique, au moyen de deux circuits de filtrage distincts pour chaque borne de photodétecteur,
- les premiers circuits de filtrage appelés filtres de polarisation (TBF, BBF), complètent les circuits de polarisation pour tous les photodétecteurs individuels, reliant, par leur intermédiaire, les bornes de photodétecteur individuel à la polarité de source appropriée, dans laquelle « appropriée » signifie qu'il s'agit de Vbias si la borne du photodétecteur est la cathode ou de GND si la borne du photodétecteur est l'anode et,
- le second circuit de filtrage, appelé filtres de charge, fonctionne comme des filtres de signaux de sortie, pour obtenir des signaux de courant individuels provenant de chaque photodétecteur individuel, représentant la quantité de lumière capturée par chaque photodétecteur individuel dans l'agencement, donnant lieu à des signaux X**ᵢ** découplés.

2. Topologie de réseau de lecture selon la revendication 1, dans laquelle le signal temporel est sélectionné comme l'un des éléments suivants :
- la somme, au niveau de la Jonction Unique (K), de toutes les bornes de cathode individuelles,
-- la somme, au niveau de la Jonction Unique (Pzk), de toutes les bornes de cathode de photodétecteurs individuels, y compris les lignes à retard routées,
- la somme, au niveau de la Jonction Unique (A), de toutes les bornes d'anode des photodétecteurs individuels,
- la somme, au niveau de la Jonction Unique (Pzk), de toutes les bornes de cathode de photodétecteurs individuels câblées indirectement,
- la somme, au niveau de la jonction unique (A), de toutes les bornes d'anode de photodétecteurs individuels câblées indirectement.

3. Topologie de réseau de lecture selon la revendication 1, dans laquelle le signal temporel est choisi comme :
- le résultat du signal différentiel entre la Jonction Unique (K) de toutes les bornes de cathode des photodétecteurs individuels et la Jonction Unique (Pza) de toutes les bornes d'anode des photodétecteurs individuels reliées indirectement, ou
- le résultat du signal différentiel entre la Jonction Unique (A) de toutes les bornes anodiques de photodétecteurs individuels et la Jonction Unique (Pzk) de toutes les bornes de cathode de photodétecteurs individuels câblées indirectement.

4. Topologie de réseau de lecture selon l'une quelconque des revendications précédentes, dans laquelle les photodétecteurs individuels sont des SiPM.

5. Topologie de réseau de lecture de niveau supérieur pour la tomographie par émission de positrons, PET, comprenant une pluralité de réseaux de lecture de niveau inférieur, compris dans la topologie de réseau de lecture définie dans l'une quelconque des revendications 1 à 4.

6. Topologie de réseau de lecture de niveau supérieur selon la revendication 5, avec p rangées et q colonnes, comprenant :
un nombre q de réseaux de lecture de niveau inférieur, chacun ayant une seule colonne et un nombre p de rangées, ou
- un nombre p de réseaux de lecture de niveau inférieur, chacun ayant une seule ligne et un nombre de colonnes q.

7. Module de détecteur nucléaire comprenant une ou plusieurs topologies de réseau de lecture telles que celles définies dans l'une quelconque des revendications 1 à 6.

8. Module de détecteur nucléaire selon la revendication 7, dans lequel une pluralité de réseaux de lecture sont agencés, qui sont choisis comme :
- une colonne, et un nombre p de lignes, ou
- une ligne, et un nombre q de colonnes,
de telle sorte que les différents signaux de référence temporelle obtenus à partir de chaque réseau de lecture sont reliés à un schéma de lecture de charge pour obtenir des informations d'énergie et de position par rapport à la pluralité de réseaux de lecture.

9. Module de détecteur nucléaire selon la revendication 8, constitué d'un nombre q de réseaux de lecture agencés en colonnes, dont chacun comporte une colonne, et d'un nombre p de rangées, de telle sorte que les différents signaux de référence temporelle q obtenus à partir de chaque réseau de lecture sont reliés à un schéma de lecture de charge pour obtenir des informations d'énergie et de position par rapport aux colonnes de la pluralité de réseaux de lecture, tandis que les différents signaux de charge p Xi sont reliés à un schéma de lecture de charges pour obtenir des informations d'énergie et de position par rapport aux rangées de la pluralité de réseaux de lecture.

10. Module de détecteur nucléaire selon la revendication 8, constitué d'un nombre q de réseaux de lecture agencés en rangées, dont chacun a une rangée, et d'un nombre q de colonnes, de telle sorte que les différents signaux de référence temporelle p obtenus à partir de chaque réseau de lecture sont reliés à un schéma de lecture de charge pour obtenir des informations d'énergie et de position par rapport aux rangées de la pluralité de réseaux de lecture, tandis que les différents signaux de charge q Xi sont reliés à un schéma de lecture de charges pour obtenir des informations d'énergie et de position par rapport aux colonnes de la pluralité de réseaux de lecture.

11. Module de détecteur nucléaire selon l'une quelconque des revendications 7, 8, 9 ou 10, comprenant en outre :
- une partie en cristal scintillateur faisant face au photodétecteur,
- un boîtier pour protéger le module de la lumière extérieure et des contraintes mécaniques,
- une électronique de conversion analogique-numérique capable de traiter tous les signaux de positionnement et d'énergie obtenus, et de les transmettre par l'intermédiaire d'une interface informatique à haut débit pour qu'ils soient traités à la volée, ou stockés sur un disque informatique pour être traités ultérieurement, ainsi que de conserver le signal temporel.

12. Système ToF-PET comprenant
- une pluralité de modules détecteurs tels que ceux décrits dans l'une quelconque des revendications 7 à 11, agencés dans un portique mécanique, ayant une configuration symétrique, tous les modules entourant et faisant face au volume de détection pouvant être couvert par ceux-ci,
- un module électronique de traitement de coïncidences capable de traiter les signaux temporels provenant d'une pluralité de modules tels que décrits dans la revendication 11 pour fournir un appariement approprié entre de multiples combinaisons de modules, permettant de traiter efficacement sous forme d'un bloc unique tous les signaux relatifs à tout événement PET individuel, quel que soit le module dont ils proviennent, ainsi que capable de numériser et de transmettre tous les signaux liés au temps à travers une interface informatique à haute vitesse pour être traités à la volée, ou stockés sur un disque informatique pour être traités ultérieurement, y compris les informations de temps de vol de tous les événements détectés.

13. Système ToF-PET selon la revendication 12, comprenant en outre un module logiciel capable de traiter toutes les données stockées dans l'ordinateur ou reçues en temps réel, pour fournir des images ToF-PET de haute qualité, à l'aide d'un quelconque ordinateur ou interface numérique.

14. Procédé d'obtention d'informations de temps et de position à partir des signaux provenant d'un photodétecteur ToF-PET, dans un module de détection d'un dispositif PET, le procédé comprenant les étapes comprenant
- l'agencement d'un cristal de scintillation dans un module détecteur
- l'agencement d'une topologie de réseau de lecture telle que celle définie dans l'une quelconque des revendications 1 à 12,
- l'entrée d'un rayon gamma dans le module et l'impact sur le volume du cristal scintillateur, créant un faisceau lumineux qui se propage partout, y compris dans la zone de photodétecteur de réseau de lecture,
- l'entrée du faisceau lumineux provenant d'un cristal scintillateur dans la zone de photodétecteur de réseau de lecture,
- la conversion du faisceau lumineux en une distribution étalée de signaux électriques provenant de l'ensemble de la zone de photodétecteur,
- l'acheminement du premier signal détecté depuis une partie quelconque du photodétecteur vers un amplificateur rapide pour le désigner comme signal temporel de sortie, représentant l'instant exact de l'arrivée du rayon gamma dans le volume de cristal scintillateur,
- l'acheminement de chacun des signaux électriques provenant de l'ensemble de la zone de photodétecteur, au moyen de filtres de charge, pour fournir des sorties de signal de charge découplés compatibles avec des circuits électroniques de lecture couramment utilisés pour extraire la position et l'énergie du rayon gamma.

15. Procédé selon la revendication 14, qui comprend en outre la manipulation de données provenant d'au moins deux modules, fournissant des informations selon la revendication 14, obtenant les lignes de réponse ToF nécessaires pour créer une image ToF-PET.
